(19) **Europäisches Patentamt / European Patent Office / Office européen des brevets**

(11) **EP 3 474 801 B1**

(12) # EUROPEAN PATENT SPECIFICATION

(45) Date of publication and mention
of the grant of the patent:
**24.01.2024 Bulletin 2024/04**

(21) Application number: **17734216.9**

(22) Date of filing: **23.06.2017**

(51) International Patent Classification (IPC):
**A61F 13/551** (2006.01)    **A61F 13/26** (2006.01)
**A61F 13/34** (2006.01)

(52) Cooperative Patent Classification (CPC):
**A61F 13/55185; A61F 13/266; A61F 13/34**

(86) International application number:
**PCT/US2017/039092**

(87) International publication number:
**WO 2017/223500 (28.12.2017 Gazette 2017/52)**

(54) **PACKAGE ASSEMBLY FOR OR WITH A TAMPON APPLICATOR**

VERPACKUNGSANORDNUNG FÜR ODER MIT EINEM TAMPONAPPLIKATOR

ENSEMBLE D'EMBALLAGE POUR OU AVEC UN APPLICATEUR DE TAMPON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.06.2016  US 201615191647
24.06.2016  EP 16176207**

(43) Date of publication of application:
**01.05.2019  Bulletin 2019/18**

(73) Proprietor: **Edgewell Personal Care Brands, LLC
Chesterfield, MO 63017 (US)**

(72) Inventors:
• **BUELL, Sezen
  Shelton, Connecticut 06484 (US)**
• **DEOLIVEIRA, Ricardo
  Allendale, New Jersey 07410 (US)**
• **TIMMERS, Richard
  Allendale, New Jersey 07401 (US)**
• **NIGAM, Pankaj
  Allendale, New Jersey 07401 (US)**
• **ABDUL, Adeyinka
  Dover, Delaware 19904 (US)**

• **BURKHARDT, Philip
  Dover, Delaware 19904 (US)**
• **OSIECKI, Scott
  Shelton, Connecticut 06484 (US)**
• **HILLEGASS, Kyle
  Shelton, Connecticut 06484 (US)**
• **FEDORA, Joseph
  Shelton, Connecticut 06484 (US)**
• **MOHAMED, Hassan
  Allendale, New Jersey 07401 (US)**
• **PREISNER, Peter
  deceased (US)**

(74) Representative: **dompatent von Kreisler Selting Werner -
Partnerschaft von Patent- und Rechtsanwälten mbB
Deichmannhaus am Dom
Bahnhofsvorplatz 1
50667 Köln (DE)**

(56) References cited:
| | |
|---|---|
| WO-A1-01/19310 | WO-A1-2010/120282 |
| WO-A1-2014/004560 | WO-A1-2017/015426 |
| US-A- 5 988 386 | US-A1- 2016 008 118 |

**Description**

BACKGROUND OF THE DISCLOSURE

1. Field of the Disclosure

The present disclosure relates to a package assembly for or with a tampon applicator or other feminine hygiene device. More particularly, the present disclosure relates to a cap for a tampon applicator and, in particular, a cap for a tampon applicator that eliminates the need for separate packaging and also, preferably, functions to disguise the tampon from recognition as a tampon, both before and after use, and is durable.

2. Description of Related Art

Feminine hygiene devices, and other devices for insertion into the body, such as, for example, tampons, pessaries, suppositories and other vaginal insertion devices are used, for example, by women within the vagina for feminine needs, such as, for example, to absorb menstrual flow. Many women almost always carry a feminine hygiene product with them, i.e., in their purse, in their pockets or in other quickly accessible locations, since the beginning of menstrual flow can be unexpected.

Current tampons are typically individually packaged in wrappers that are usually made of plastic film, such as polypropylene and polyethylene, or paper. It is important for this package to stay intact as tampons can get soiled by dust, upon coming in contact with other objects, unintended touching and the like.

Using dirty and damaged tampons can result in injury, infection or illness. Currently available wrappers tend to tear, break open and/or puncture when they travel in the purse or pocket for an extended amount of time. The exposed, and possibly damaged, tampons may cause infection and can result in pain and discomfort during insertion. It is also very inconvenient to the user as she may not be able to use the product and have to find an alternative.

Another drawback of the current wrappers is that the shapes are very traditional and colors of the wrappers are either very feminine or very vibrant, making it very easy to identify tampons from a distance. This can be embarrassing for some women, especially a teenager who is just starting to have menstrual cycles.

Yet another drawback of the current tampon and wrapper assemblies is the number of steps needed to remove the tampon from the wrapper and thereafter insert the tampon, and dispose of one or more or all of the used tampon, applicator, wrapper, and other hygiene product. For instance, current tampon and wrapper assemblies require (1) tearing or opening the wrapper, typically involving two hands (i.e. one hand to grip a first portion of the wrapper and the other hand to move another portion of the wrapper in an opposite direction from the first edge, thereby causing the wrapper to tear, break or open), (2) removing the tampon from the wrapper, also typically requiring two hands (i.e. one hand to hold the opened wrapper and the other hand to remove the tampon from the wrapper), (3) positioning the tampon applicator for use, which may also typically require two hands (i.e. reorienting the tampon in one's hand for proper insertion orientation, withdrawing the plunger (with one hand) from the applicator (by holding the applicator in the other hand) such that the tampon applicator is positioned and ready to apply a force to the pledget in the applicator for insertion into the body, putting the plunger, with one hand, into the applicator (being held by the other hand) such that the plunger can be used to apply a force to the pledget in the applicator for insertion into the body), (4) inserting the tampon into the body, (5) removing the applicator from the body (for systems employing an applicator), (6) re-opening the wrapper for placement of the used tampon and/or applicator, which also requires two hands: one hand to hold the used tampon and the other to hold the wrapper open, (7) placing the used tampon and/or applicator into the opened wrapper, which also requires to hands: one hand to hold the used tampon and the other to hold the wrapper open, and (8) disposing of the wrapper containing the used tampon and/or applicator. Accordingly, current wrapper and tampon assemblies typically require five (5) to six (6) steps to open, prepare, insert the tampon, and remove the used tampon and/or applicator, and seven (7) to eight (8) steps if including the steps of disposing of the used tampon. Further, many of these steps require the use of two hands.

Yet another drawback of the current tampon and wrapper assemblies is the difficulty of disposal. Since the wrapper does not tear consistently, it makes it very difficult to place the used applicator back into the torn wrapper. In most instances, the user needs to find alternative ways for disposal such as wrapping the used applicator with toilet paper and/or disposing the applicator and wrapper separately.

Yet another drawback of the current wrappers is that they are bulky, especially for full-sized applicators, and thus occupy a rather large amount of space. The larger size also makes the wrapper difficult and indiscrete to carry about or carry to the bathroom as the wrapper does not fit in the palm of the user's hand.

Therefore, it has been determined that there is a need for a tampon applicator and package assembly that will provide small, durable packaging and provide discrete user experience.

A package assembly for and/or with a tampon applicator is described in WO-A-2014/004560. From WO-A-2017/015426 (EP-A-3 324 909, document according to Article 54 (3) EPC), WO-A-2010/120282, WO-A-01/19310, and US-A-5 988 386 divers tampon applicators are known.

SUMMARY OF THE DISCLOSURE

The present invention provides a tampon applicator package assembly with a tampon assembly having the features of claim 1. Diverse embodiments of the invention are the subject matter of the respective dependent claims.

The present disclosure provides a tampon applicator package assembly that includes a cap and a tampon applicator having a barrel and a plunger. The tampon applicator has a barrel with an insertion tip and a plunger receiving end opposite the insertion tip. The plunger has a first end in the barrel and a second end, opposite the first end, at least partially outside of the barrel. The cap has a cap wall that encloses the barrel and connects to the second end of the plunger or the plunger receiving end of the barrel.

In one embodiment, the present disclosure provides a tampon applicator package assembly comprising a cap and a tampon applicator having a barrel and a plunger, with the tampon applicator disposed in the cap. The plunger has an end portion that mates with the cap to provide an at least partial seal that completely encloses the barrel and the plunger in the cap.

In another embodiment, the present disclosure provides a tampon applicator package assembly comprising a tampon applicator having a barrel and a plunger with a grippable area, a cap having a closed end and an open end, and a cap for sealing the open end of the cap. The tampon applicator is disposed in the cap. The cap cover has an inner end and an outer end. The inner end is disposed proximal the tampon applicator. When the cap cover is placed on the open end of the cap, an at least partial seal is provided with the cap that completely encloses the barrel and the plunger. When the cap cover is removed from the open end of the cap, the grippable area of the plunger is exposed.

In still another embodiment, the present disclosure provides a tampon applicator package assembly comprising a tampon applicator having a barrel and a plunger with a grippable area, a cap having a closed end and an open end, and a cap cover for placing over and sealing the open end of the cap. The tampon applicator is disposed in the cap. The cap cover has an inner end and an outer end. The inner end is disposed proximal the tampon applicator. When the cap cover is placed on the open end of the cap, an at least partial seal is provided with the cap that completely encloses the tampon applicator and the cap cover matingly engages at or near the grippable area of the plunger. When the cap cover is removed from the open end of the cap, the grippable area is withdrawn at least partially from the cap.

In yet another embodiment, the tampon package assembly further comprises a string attached to the pledget in the tampon applicator that is enclosed in the tampon applicator package. When the cap cover is removed from the open end of the cap, the string is exposed.

In a further embodiment, the tampon package assembly comprises a string attached to the pledget and the string is folded and enclosed in the tampon applicator package when the cap cover is placed on the cap. When the cap cover is removed from the cap, the string is at least partially unfolded. The string may be at least partially unfold by attachment to the cap cover by, e.g., mechanical attachment such as through the use of adhesive, or by other physical attachment to the cap cover.

In a still further embodiment, the tampon package assembly has the string attached to the pledget and affixed to the plunger, and the string is immediately exposed when the cap cover is removed.

In an additional embodiment, the cap and/or the cap cover is made of a foil or paper (or other flexible material). The cap cover and is peelable away from the cap to expose the plunger or grippable area of the plunger. The foil or paper (or other flexible material) may be peelable away from the open end of the cap due to attachment by an adhesive or through the use of perforations. In a further additional embodiment, the cap can be a flexible material and the cap cover can be a more rigid material. The cover can at least partially connect to either the cap, the grippable area of the plunger, or both.

In a further embodiment, the cap has two covers, one disposed at or near the pledget-containing end of the barrel and one disposed at or near the grippable area of the plunger.

In a still further embodiment, the tampon applicator package assembly has two caps. In a yet further embodiment, the two caps can be at least partially attached by a connector and/or ring.

In another embodiment, a tampon applicator package assembly enables the user to open, prepare, insert, and remove the tampon applicator in four steps. In a further embodiment, a tampon applicator package assembly enables the user to dispose of the tampon applicator package assembly in one further step.

In a yet further embodiment, a tampon applicator package assembly that has increased durability is provided, so that the tampon applicator package assembly cannot be torn or punctured during a storage position at any time including while being transported.

The above-described and other features and advantages of the present disclosure will be appreciated and understood by those skilled in the art from the following detailed description, drawings, and appended claims.

BRIEF DESCRIPTION OF THE DRAWINGS

FIG. 1A is an exploded top view of an exemplary embodiment of a tampon applicator package assembly of the present disclosure with a tampon applicator having a plunger in a use position and a cap disconnected from the tampon applicator.

FIG. 1B is an exploded perspective view of the tampon applicator package assembly of FIG. 1A.

FIG. 1C is an exploded perspective view of the tampon applicator package assembly of FIG. 1A having the plunger in the stored position and the cap disconnected from the tampon applicator.

FIG. 1D is an inverted top view of the cap of FIG. 1A.

FIG. 2 is a cutaway perspective view of the tampon applicator package assembly of FIG. 1A having the plunger in the stored position and the cap connected to the tampon applicator.

3A-3O illustrate various views of tampon assemblies and components incorporating a slit-surface.

FIG. 4A-4B illustrate tampon assemblies including a mechanism for retaining a string.

FIG. 5A-E illustrate tampon assemblies including a cap and a barrel configured to manage a string.

FIG. 6 illustrates a portion of a barrel including petals.

FIGS. 7A-B illustrate tampon applicator package assemblies including a flip top cover configured to accommodate a slit-surface structure and a string reservoir.

FIGS. 8A-D illustrate tampon assemblies that include an obstruction for managing a string.

FIGS. 9A-F illustrate various designs and geometries for an obstruction.

FIGS. 10A-D illustrate tampon assemblies that include a sticky tab for managing a string.

FIGS. 11A-D illustrate tampon assemblies that include tabs for managing a string.

FIGS. 12A-D illustrate various designs and geometries for a plunger for managing a string.

FIGS. 13A-D illustrate a tampon package applicator assembly according to the invention in various stages of deployment having retention structures.

FIGS. 14A-B illustrate a tampon package applicator assembly.

FIGS. 15A-B illustrate a tampon package applicator assembly having retention features.

FIGS. 16A-C illustrate a tampon package applicator assembly having retention features.

DETAILED DESCRIPTION OF THE DISCLOSURE

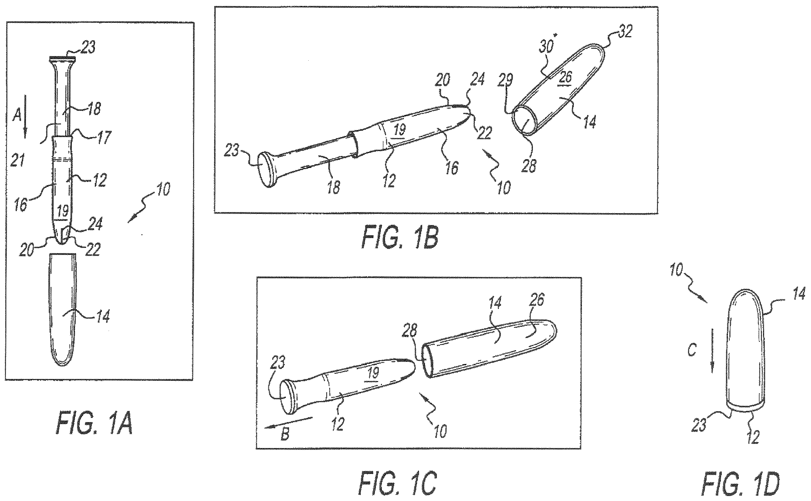

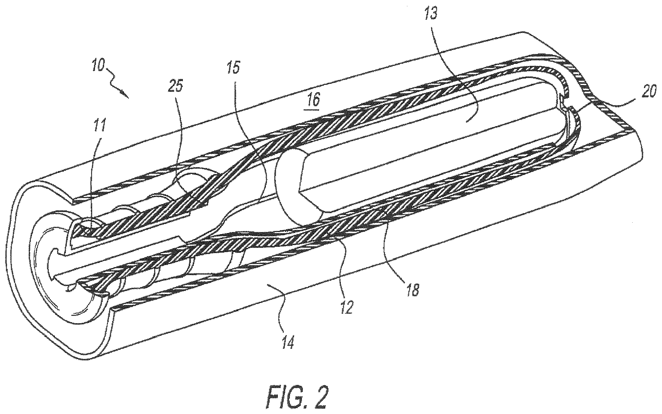

Referring to the drawings and in particular to FIGS. 1A-1D, an exemplary embodiment of a tampon applicator package assembly according to the present disclosure is shown and generally referred to by reference numeral 10. The terms "tampon applicator package assembly" and "tampon package applicator assembly" are synonymous and interchangeable for purposes of the present disclosure, and are both generally referred to by reference numeral 10 and/or 2010. Tampon applicator package assembly 10 has a tampon applicator 12 and a cap 14. Tampon applicator 12 is shown in a use position in FIG. 1. Tampon applicator 12 has a barrel 16 for housing a pledget 13 (shown in FIG. 2) and a plunger 18 in a telescoping configuration. Pledget 13 is an absorbent material. Barrel 16 has a barrel wall 19 with an insertion tip 20. Insertion tip 20 has a plurality of petals 22 separated by cuts 24 through barrel wall 19. Plunger 18 has a telescoping portion 21 and an end portion 23. End portion 23 may be a cap cover that connects to a remainder of plunger 18. As with tampon applicators, barrel 16 can be inserted into a body of a user to eject the pledget into the body, such as, for example, to absorb menstrual flow. When barrel 16 is inserted into the user's body, the user applies a force to end portion 23 of plunger 18 to move telescoping portion 21 of plunger 18 in a direction shown by arrow A further into barrel 16 that moves the pledget through the opening and, thus, through insertion tip 20 into the body.

Referring to FIG. 1B, cap 14 has a cap body 26 with a cap opening 29 and a cavity 28 in cap body 26. Cap 14 is sized and shaped to cover barrel 16. For example, cap body 26 has a cylindrical wall 30 and a top portion 32 that has a similar or mating shape to insertion tip 20.

Referring to FIG. 1C, tampon applicator package assembly 10 is shown with cap 14 disconnected from tampon applicator 12. Tampon applicator 12 has plunger 18 in a stored position. Prior to use, telescoping portion 21 of plunger 18 and pledget 13 (FIG. 2) are housed in barrel 16. Plunger 18 is moved to the use position by applying a force to end portion 23 in a direction shown by arrow B in FIG. 1C moving end portion 23 away from barrel 16. When plunger 18 is in the use position, plunger 18 is adjacent pledget 13 and the user can apply the force to end portion 23 to move telescoping portion 21 in the direction shown by arrow A in FIG. 1A to move the pledget through insertion tip 20 into the body of the user. In another embodiment, cap 14 may be connected to a plunger receiving end 17 instead of end portion 23 of plunger 18. In this latter embodiment, plunger receiving end 17 and/or cap 14 will be provided with one or more structures 11. "Structures" is hereinafter defined to include, without limitation, one or more protrusions, slits, ridges or other type structure(s) that will, amongst other things, become apparent to one skilled in the art based upon the disclosure that follows. The Structures provide plunger receiving end 17 and cap 14 with an at least partial mating engagement. "Mating engagement" is hereinafter defined to include, without limitation, a friction-fit, snap-fit, button-fit, a detent, a threaded connection, a bayonet connection, and other connections, or any combinations thereof that will become apparent to one skilled on the art based upon the disclosure that follows.

Referring to FIG. 1D, tampon applicator package assembly 10 has tampon applicator 12 connected to cap 14 and plunger 18 in the stored position. End portion 23 of plunger 18 may be sized so that, when cap 14 is connected to tampon applicator 12, at least a portion of end portion 23 is flush with cap 14. Tampon applicator 12 is connected to cap 14 so that barrel 16 is housed in cap 14. For example, cap 14 and end portion 23 are sized to form a friction fit, or end portion 23 has groove and cap 14 has a Structure that fits in the groove forming a snap fit so that a predetermined force applied by user to end portion 23 in a direction shown by arrow C disconnects cap 14 from end portion 23.

Referring to FIG. 2, plunger 18 may have a slit 15 on one side or both sides to enclose a pledget 13 in barrel 16. In other embodiments, there may be a plurality of slits 15 along at least a portion of the length of the plunger 18 such that the plunger 18 with a plurality of slits 15 encloses pledget 13 in barrel 16. In other words, plunger 18 may have one or more slits 15 along at least a portion of the length of the plunger such that the plunger 18 at least partially encloses pledget 13, where the plunger is enclosed within barrel 16. Pledget 13 can be inserted through insertion tip 20 urging and opening plunger 18 to open along slit 15. Alternatively, pledget 13 can be inserted through insertion tip 20 and thereafter plunger 18 can be forced to open around pledget 13 via one or more slits 15. Structure 11 can further function as a stopping mechanism 25, that may be shaped similar to a shelf, can be added to barrel 16 to keep pledget 13 forward when plunger 18 is pulled out of barrel 16 to the use position. Plunger 18 shown in FIG. 2 may have end portion 23 as shown in FIG. 1A. There are alternative stopping mechanisms, such as ridges inside the barrel 16 or outside the plunger, to keep pledget 13 in place until the user deploys the plunger 18 as desired. Once plunger 18 is pulled out, there are other stopping mechanisms 25, such as inside ridges or shelves on the inner surface of barrel 16 or outside ridges or shelves on the outer surface of the plunger 18, to keep the plunger 18 from disengaging from the barrel 16.

Referring again to FIGS. 1A-1D, plunger 18 can be moved from the stored position to the use position once cap 14 is at least partially disconnected from tampon applicator 12, or while tampon applicator 12 is still connected to cap 14. Once cap 14 is disconnected from tampon applicator 12 and plunger 18 is moved from the stored position to the use position, tampon applicator 12 can be used to insert pledget 13 into the body of the user. After pledget 13 is inserted, the user may re-connect cap 14 to tampon applicator 18 to form the same configuration as prior to use for disposal of tampon applicator 12 and cap 14.

The present disclosure provides the user the advantage of removing the tampon applicator package assembly 10 from a stored position to a use position with virtually one hand and/or in a minimal number of actions. For instance, the tampon package applicator assembly 10 can be held and opened in one hand of the user (see the embodiments described throughout the present disclosure for ways of opening cap 14). Once the cap 14 is opened to reveal at least the end portion 23 of plunger 18, the user can grasp or touch the end portion 23 of plunger 18 to enable the telescoping portion 21 of plunger 18 to be at least partially exposed from the barrel 16 that, in turn, is at least partially contained in cap 14. Once the plunger 18 is sufficiently exposed so that the plunger 18 is still at least partially contained in tampon applicator 12 but oriented so that plunger 18 is adjacent the pledget 13, tampon applicator 12 and plunger 18 are in a prepared position. The user can remove the tampon applicator 12 and plunger 18 in the prepared position and thereafter insert the tampon applicator 12 containing pledget 13 into the user's body. The user can accomplish the acts of preparing the tampon applicator 12 and plunger 18 and removing tampon applicator 12 and plunger 18 from cap 14 in a single motion, and thus in just one step. The user can thereafter remove the tampon applicator 12 from the body. In a further embodiment, the user can thereafter, with one hand and with a minimal number of steps or actions, place the used tampon applicator back into cap 14. In another embodiment, a soiled feminine hygiene product or other hygiene product such as, for example, another tampon, a wipe, a pledget, pessaries and/or suppository can also be placed into the cap 14 for discreet and/or hygienic disposal. In further embodiments comprising one or more cap covers 26, the cap cover 26 that is at least partially disengaged from the cap 14 can be replaced thereby further containing the soiled article for more discreet and/or more hygienic disposal.

In other words, the user can accomplish the task of opening the tampon applicator package assembly 10, preparing the tampon applicator 12 for insertion, inserting the tampon applicator 12 into the body, and removing the used tampon applicator from the body in just four (4) steps. Further, the user can thereafter dispose of the tampon and/or tampon applicator 12 in just two additional steps for a total of six (6) steps. For instance and as described throughout the present disclosure, once the tampon package applicator assembly 10 is at least partially opened, the user can remove the tampon applicator 12 so that it is removed and prepared for insertion into the body in one further step; this avoids the user having to prepare the pledget 13 and/or tampon applicator 12 in several steps. Additionally, due to the present disclosure's tampon applicator package assembly 10, the used tampon applicator 12 can be, in one step, easily replaced into the vacated tampon applicator package assembly 10 (thereby eliminating the need of the prior art wrappers of re-opening and/or holding the wrappers open in order to permit replacing the used tampon and/or tampon applicator into the vacated prior art wrapper). As such, the present disclosure's tampon applicator package assembly 10 can reduce the number of steps or actions the user must accomplish in order to utilize a tampon. Furthermore, this utilization is accomplished virtually or almost solely with one hand.

Cap 14, amongst other components of tampon applicator package assembly 10, can be made from one or more plastics, such as polyethylene, polypropylene, polybutylene, polystyrene, polyvinylchloride, polyacrylate, polymethacrylate, polyacrylonitrile, polyacrylamide, polyamide, nylon, polyimide, polyester, polycarbonate, polylactic acid, polyhyroxyalkanonate, ethylene vinyl acetate, polyurethane, silicone, thermoplastic elastomers, thermoplastic starch, t-polyisoprene, derivatives thereof, copolymers thereof, mixtures thereof, or any other suitable plastic material. Alternatively, cap 14, amongst other components of tampon applicator package assembly 10, can be made from a non-polymer material such as paper, paperboard, cardboard, cellulose (such as molded cellulose), metal, textile, wood or any combination thereof. Cap 14, amongst other components of tampon applicator package assembly 10, can be laminated, as opposed to single layer. In certain embodiments, additives can be included to alter or enhance certain material properties. Suitable additives include, but are not limited to, one or more mold release agents, anti-slip agents, surface energy modifiers, non-organic fillers, and/or any other suitable additives, or any combination thereof. Cap 14, amongst other components of tampon applicator package assembly 10, can have different coatings applied thereto for ease of use and decorating purposes. The material of cap 14, amongst other components of tampon applicator package assembly 10, can be biodegradable, compostable or flushable. The material of cap 14, amongst other components of tampon applicator package assembly 10, can be made using various manufacturing techniques including, but not limited to, injection molding, extrusion molding, blow forming and thermoforming, injection blow molding, rotational molding, transfer molding, co-molding, over-molding, casting, calendaring, coating, compression molding, laminating, pultrusion, slush molding, transfer molding, contact molding, metal forming processes and dipping, or any combination thereof. Barrel 16, plunger 18 and cap 14 can be made from different materials, and/or can be made up of different colors. A cross-sectional shape of cap 14 can be, without limitation, round having a constant or changing diameter, semicircular, rectangular, polyhedral, prismatic, triangular, may have a perimeter that is undulating, toothed or other geometry, or any combination of any of the foregoing. Cap 14 can be shaped so that it prevents tampon applicator package assembly 10 from rolling when placed horizontally. Cap 14 can be opaque or transparent, can be printed/decorated, and can be manufactured with relief surfaces to control and improve the visual appearance and appeal, or any combinations of the foregoing. Cap 14 can be decorated using several different methods including, but not limited to, in-mold texture change, printing, in-mold labeling, heat transfer foil, applying a printed label, embossing, etching, shrink wrapping, selective polymer addition, co-molding, bleaching, over-molding, or any combination thereof. The shape and decoration (or look) on cap 14 can be chosen obfuscate that it is a tampon. For example, cap 14 can have the look of a cosmetic object, such as a lipstick or of any ordinary object, such as, but not limited to, a flashlight or a lighter. One skilled in the art understands that the present disclosure is directed to a tampon package applicator assembly 10 whose aesthetics and/or otherwise visual appearance are other than that of a tampon package. In other words, the look of tampon applicator package assembly 10 serves to alleviate or eliminate the apprehension or embarrassment felt by the user due to being seen with a tampon by another. Also, all variations of cap 14 can look shiny, matte, contain a graphic or image, or a combination thereof, to further the desired aesthetics.

It will be appreciated by those of ordinary skill in the art that tampon applicator package assembly 10 may be provided with two cap covers, each independently sealing an open end of a cap. In embodiments, including but not limited to, with two caps and/or two covers, with two caps and one cover, with one cap and two covers, the caps and cap covers can be of any type as described in the present disclosure, and any combinations thereof. In this embodiment, the cap will be provided with two open ends, one disposed proximal to the plunger end of the tampon applicator ("bottom end") and one disposed proximal the pledget-containing end of the barrel ("top end"). A cap can have any of the cap and cap cover configurations hereinbefore described, such as a peelable cap cover or perforated cap cover at each open end of the cap, can have a flip top cap cover at each open end of the cap, or can have an at least partial mating engagement between cap and one or more of the cap covers at each open end of the cap. Of course, where the cap is provided with two open ends, each open end can be provided with another cap and/or cap cover. This configuration of tampon applicator package assembly 10 has a benefit of ease of manufacture. In this regard, the barrel can be inserted into the cap via the "top" opening, and the plunger/pledget combination can be inserted into the cap via the "bottom" opening. This "two" insertion assembly provides for easier insertion of the barrel because, in this instance, the barrel does not need to be pushed past any Structure (if present) disposed on the inner surface of the cap. Although not preferred, it is also possible to insert the complete tampon applicator including the barrel, the plunger and the pledget into the cap through the "top" opening.

Currently available tampon applicator wrappers tear easily during transportation. The present disclosure resolves this problem by eliminating the wrapper and replacing it with a cap and/or cap cover, including, but not limited to, cap 14. Cap 14 is made from a material that has greater durability. For example, the cap of the present disclosure is fashioned so that it avoids puncturing, tearing and/or other damaging that renders the contents of the tampon applicator package assembly vulnerable to contamination and/or destruction. In sum, it has been found that another advantage of present tampon applicator package assemblies is that disposal of used tampon applicators is sanitary and discrete. For purposes of this paragraph, all embodiments of the disclosed tampon applicator assemblies will be referred to as tampon applicator package assembly 10. Traditional wrappers need to be torn to remove the applicator therefrom. It is usually difficult, if not impossible, to place the used tampon applicator back into the torn wrapper. In the present disclosure, cap 14 is one resilient piece, and it is very easy to place a used tampon applicator back in cap 14. Once discarded, only cap 14 can be seen, and cap 14 does not resemble a used tampon applicator. Furthermore, the soiled or discarded feminine hygiene product(s) is contained in cap 14, which enables a more hygienic disposal, thereby diminishing and/or preventing the opportunity for the used applicator or other soiled/ discarded feminine hygiene item from coming into contact with anything other object aside from cap 14. Also, tampon applicator package assembly 10 enables a design that prevents overall apprehension or embarrassment of the user that can result from the use of tampons. Thus, the small, discrete design ensures an embarrassment- or apprehension-free usage experience. Tampon applicator package assembly 10 resembles any Personal Item that the user might carry and, thus, enables a worry-free carrying, and/or use, and/or disposal experience, and combinations thereof. Also, the variability of shapes and designs for cap 14 enables a new, fun way to use tampons.

All embodiments of the tampon applicator package assembly referred to and described above can be sized and designed to fit a full-size tampon applicator and/or a compact tampon applicator, and/or other complementary products, or any combinations thereof.

A case associated with the tampon applicator package assembly may be strong, rigid, and durable. Such a feature may be contrasted with wrappers that may be subjected to wear over time.

Flexural rigidity can be defined as the resistance offered by an object while undergoing bending. Flexural rigidity, $D$, for a thin film/plate can be calculated as:

$$D = \frac{EI}{1-\mu^2} = \frac{Ebt^3}{12(1-\mu^2)}$$

where $E$ is Young's modulus, $I$ is second moment of inertia and $\mu$ is Poisson's ratio. Second moment of inertia, $I$, for a thin film may be calculated as:

$$I_{thin\,film} = bt^3$$

where $b$ is the width of the film and $t$ is the thickness of the film.

Conventional tampon wrappers may be made from similar materials (e.g., simple elastomeric polymers such as olefins). The Poisson's ratio, $\mu$, for these materials are similar and thus don't make an appreciable difference in the calculation of flexural rigidity, $D$.

The table below summarizes $EI$ values, which represent a measure of the flexural rigidity for given geometries/configurations, for a sampling of existing tampon wrappers (regular absorbency only), as identified by wrapper names in use:

| Wrapper Name | width (mm) | thickness (mm) | I (mm$^4$) | E(MPa) | EI (Nmm$^2$) |
|---|---|---|---|---|---|
| Sport | 30.163 | 0.076 | 1.33E-02 | 22.3 | 0.298 |
| GG | 26.988 | 0.025 | 4.42E-04 | 67.8 | 0.030 |
| Pearl | 36.513 | 0.089 | 2.57E-02 | 14.8 | 0.380 |
| Radiant | 44.450 | 0.178 | 2.50E-01 | 26 | 6.496 |
| Sleek | 41.275 | 0.058 | 8.23E-03 | 16.3 | 0.134 |

Tampon applicator package assemblies in accordance with this disclosure replace a conventional wrapper with a cap (e.g., cap 14 in Figure 1A). The flexural rigidity of this cap can be calculated from the flexural rigidity formulation for a hollow cylinder:

$$EI = E\pi \frac{\left(r_o^4 - r_i^4\right)}{4}$$

where $r_o$ is the outer radius and $r_i$ is the inner radius of the hollow cylinder. The *EI* values for some tampon applicator package assemblies in accordance with this disclosure are calculated and given in the table below for given assembly types:

| Assembly style | r$_o$ (mm) | r$_i$ (mm) | I (mm$^4$) | E (MPa) | EI (Nmm$^2$) |
|---|---|---|---|---|---|
| Flip top | 10.5 | 8.75 | 4.94E+03 | 187.6 | 9.27E+05 |
| Twist-off | 10.5 | 9.325 | 3.61E+03 | 187.6 | 6.77E+05 |

The values provided in the tables are illustrative. In some embodiments, *EI* values greater than or equal to $6.7 \times 10^5$ Nmm$^2$ or $9.2 \times 10^5$ Nmm$^2$ may be used.

The *EI* values for the case can be above 10 Nmm$^2$. For example, values above $1 \times 10^3$ Nmm$^2$ and above $1 \times 10^4$ Nmm$^2$ may be used in some embodiments.

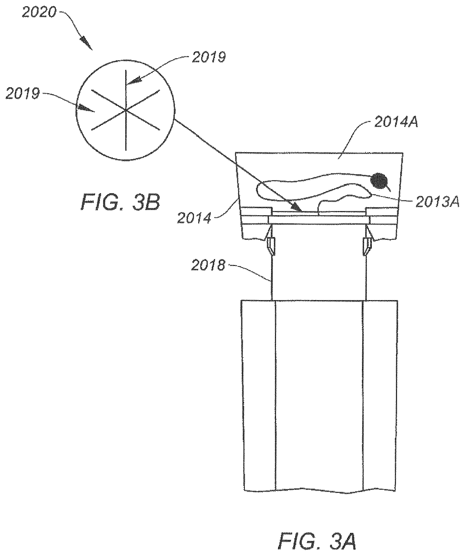

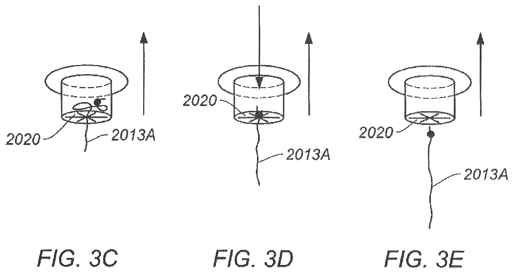

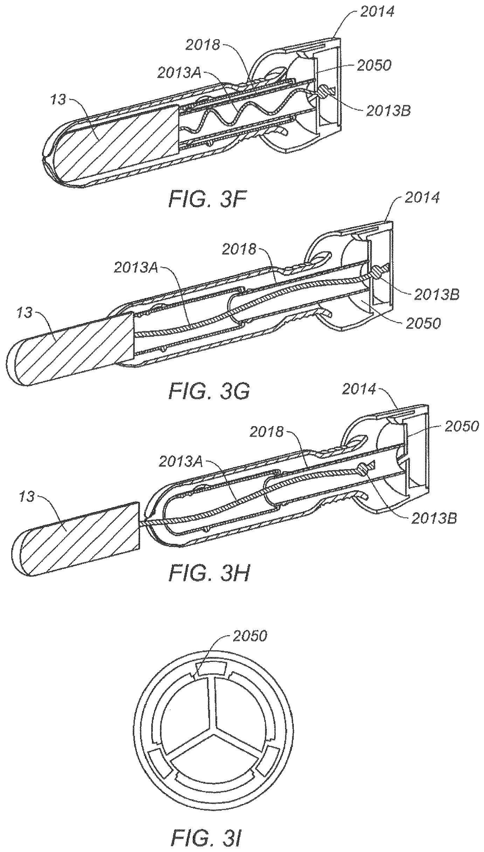

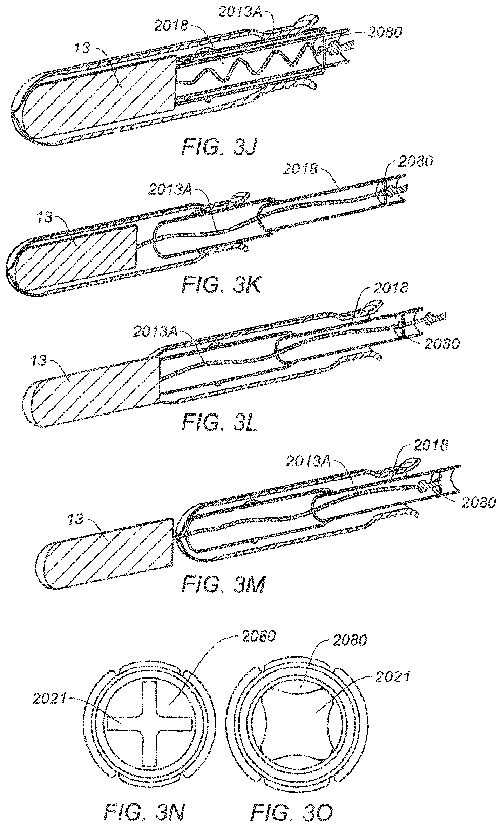

As described herein, a personal care product (e.g., pledget 13 shown in FIG. 2) accommodated by one or more tampon applicator package assemblies may include a string (e.g., string 2013A of FIG. 3D). Management of the string can prove challenging. For example, during assembly, the string must be pulled-through the plunger to ensure it is readily accessible for the consumer when the tampon applicator package assembly is opened and being prepared for deployment. For example, it may be necessary to keep the string from becoming tangled in order to promote efficiency or effectiveness in the deployment of the personal care product.

Referring to FIG. 3A, a cap 2014 (which may correspond to the cap 14) can be snapped onto an end portion of a plunger 2018 (which may correspond to the plunger 18) with friction fit, threads or any other technique of engagement. The cap 2014 may be made from one or more plastics; e.g., polyethylene, polypropylene, polybutylene, polystyrene, polyvinylchloride, polyacrylate, polymethacrylate, polyacrylonitrile, polyacrylamide, polyamide, nylon, polyimide, polyester, polycarbonate, polylactic acid, polyhyroxyalkanonate, ethylene vinyl acetate, polyurethane, silicone, thermoplastic starch, trans-polyisoprene, derivative thereof, copolymers thereof, mixtures thereof or any other suitable plastic material. Alternatively, the cap 2014 can be made from a non-polymer material such as paper, paperboard, cardboard, cellulosed (such as molded cellulose), metal, textile, wood or any combination thereof. Cap 2014 can have different coatings applied to it for ease of opening and decorating purposes. The material of cap 2014 can be biodegradable, compostable or flushable. The cap 2014 can be made using various manufacturing techniques including, but not limited to, injection molding, extrusion, blow forming, thermoforming, injection blow molding, rotational molding, transfer molding, comolding, overmolding, casting, calendaring, coating, compression molding, extrusion molding, laminating, pultrusion, slush molding, transfer molding, contact molding, metal forming processes and dipping and a combination thereof.

The cross-sectional shape of the cap 2014 can be circular, rectangular, triangle, star-shaped, flower-shaped or a combination thereof. The cap 2014 can have a built-in "slit-surface" structure 2020 having flaps 2017 separated by slits 2019. In some embodiments, structure 2020 resembles the top of a disposable coffee cup lid. Cap 2014 has a reservoir 2014A above structure 2020 to store a string 2013A when the tampon applicator package assembly is not in use. As shown in, for example, FIGS. 3N-3O, the string 2013A unwraps through a hole 2021 in the slit-surface 2020.

Referring to FIGS. 3F-3I, the slit-surface can be a separate part 2050 that can be snapped or attached to the cap 2014 in any way (ultrasonic bonding, hinge, etc.). Yet another embodiment includes a slit-surface 2080 built into the plunger 2018 as shown in FIGS. 3J-O. The location of the slit-surface 2080 can be anywhere along the length of the plunger 2018.

A slit-surface (e.g., slit-surfaces 2020, 2050, or 2080) preferably includes one to ten flaps, and in some embodiments, two to five flaps. The flaps may be thin and/or flexible such that when the string 2013A goes through the hole 2021 in the middle of the slit-surface or through the slit 2017 between the flaps 2019, they bend and give way to the string 2013A. The size of the hole 2021 in the middle may be 1% to 150% of the string 2013A knot 2013B size; in some embodiments between 20% and 70%. There might be a gap between the flaps. The gap distance between the flaps may be 1% to 150% of the string 2013A knot 2013B size; in some embodiments between 20% and 70%.

In some embodiments, the cap 2014 has a hole 2021(see FIGS. 3N-3O) to enable the string 2013A to be sucked through the plunger 2018 during assembly. The size of the hole 2021 may be between 1% and 99% of the cap 2014 diameter; in some embodiments between 20% and 80% of the cap 2014 diameter.

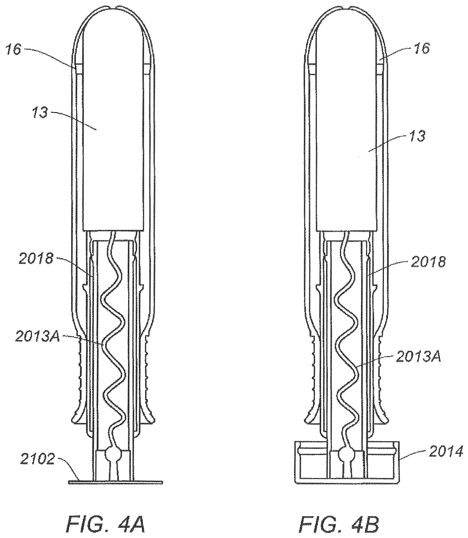

As the string 2013A is sucked through the plunger 2018, the string 2013A still needs to be enclosed within the plunger 2018 (e.g., the string 2013A may be precluded from dangling out of the plunger 2018 in some embodiments) since the cap 2014 may need to be attached to an end of the plunger 2018. FIGS. 4A-4B show an embodiment of how this can be accomplished. As the string 2013A is pulled through the plunger 2018 by a vacuum, a mechanism 2102 that would allow the vacuum/air to pass through but not the string 2013A can be placed at the end of plunger 2018. This mechanism 2102 can include a screen, sieve or any other technique that has or provides a certain pore diameter. The string 2013A can alternatively be sucked through using electrical charge/electrostatic forces such that the string 2013A can be pulled through the plunger 2018 but stopped before the string 2013A goes out of or exits the plunger 2018. This string assembly method can be applied to any of the embodiments described in this disclosure, particularly those that may require keeping the string 2013A enclosed in the plunger 2018 after the pledget 13 is inserted into the barrel 16. If the cap 2014 doesn't have a hole, the string 2013A is first sucked in then followed by the cap 2014 being snapped at an end of the plunger 2018. If the cap 2014 does have a hole 2021, then the cap 2014 can be snapped onto an end of the plunger 2018 first, then the string 2013A is sucked through the plunger 2018. Once the tampon applicator package assembly is assembled and the string 2013A is stored in the reservoir 2014A (see FIG. 3A), the hole 2021 can be covered with a label (e.g., such as sticky tab 31 in FIGS. 3C-3D) to prevent the string 2013A getting out. Alternatively, a separate piece of material can be attached to close the hole by using friction fit, threads, ultrasonic bonding, adhesives and any other joining methods available. During use, as the pledget 13 is ejected into the user's body, the string 2013A goes through the slit-surface (e.g., slit-surface 2020, 2050, or 2080) which helps the string 2013A unwrap and come out in a generally linear and/or tangle-free manner.

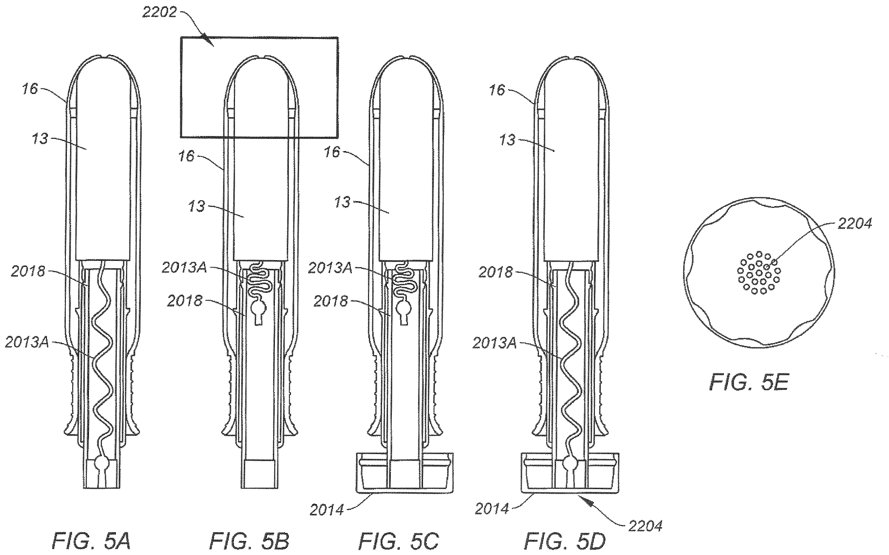

In some embodiments, the string 2013A can be sucked through the top (e.g., through the petals at the open end of the barrel 16) by a vacuum as shown via reference character 2202 in FIG. 5B. The string 2013A would then be located substantially under the pledget 13. The cap 2014 can then be attached to an end of the plunger 2018. Alternatively, the plunger 2018 and the cap 2014 can be manufactured together such that they are one piece. The cap 2014 can have a hole or several holes to allow for a vacuum to be applied. Once the cap 2014 is attached to the plunger 2018, the string 2013A can be sucked through the plunger 2018 such that the string 2013A doesn't bunch up at the bottom of the pledget 13. The application of a vacuum to the cap is reflected in FIG. 5B via reference character 2204.

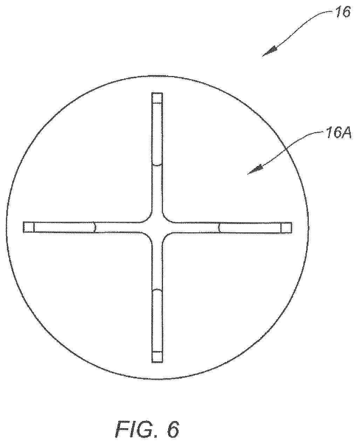

An embodiment of a portion of a barrel 16 is shown in FIG. 6. The petals 16A of the applicator can act like a slit-surface (e.g., slit-surfaces 2020, 2050, or 2080) as described above. As the pledget 13 is inserted and ejected into the user's body, the string 2013A will go through the petals 16A and unwrap as the string 2013A goes through. The petals 16A may be thin and/or flexible such that when the string 2013A goes through the hole 2021 in the middle or through the gap between the petals 16A, the petals 16A bend and give way to the string 2013A. The gap distance between the petals can be 1% to 150% of the string 2013A knot 2013B size; in some embodiments between 20% and 70%.

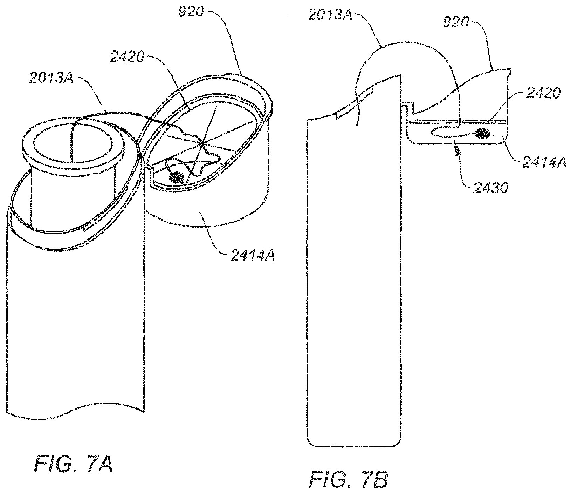

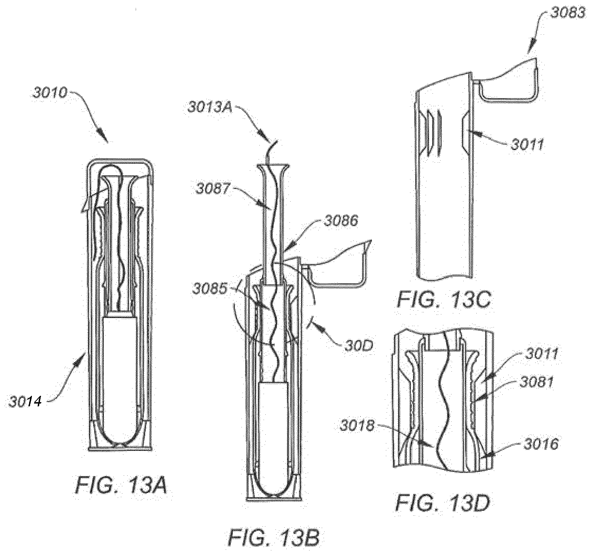

Referring to FIGS. 7A-B, a slit-surface structure 2420 and a string reservoir 2414A can be built into a flip top cover 920 as described above in connection with, e.g., FIGS. 13A-13B. Similar to above, the string goes through the hole in the middle of the slit surface. There might be a hole 2430 on the flip top cover 920 to enable the string 2013A to be sucked through the slit-surface 2420 during assembly so the string 2013A can be stored in the reservoir 2414A. Similarly, once the package is assembled and the string 2013A is stored in the reservoir 2414A, the hole 2430 can be covered with a label or a separate piece of material to prevent the string 2013A getting out. In this flip top embodiment, the string 2013A can be stored in other ways that don't require a slit-surface 2420 and reservoir 2414A. For example, the string 2013A can be wrapped around at lease a portion of plunger 18 at the end portion 23 (FIG. 1A) of the plunger 18. Alternatively, the string 2013A can be tucked in the flip top cap cover 920 (FIG. 7A) and stored until use. When the user opens the flip top cap 920, the string 2013A may be visible and readily available for the user. Yet another alternative is to lay the string 2013A along the length of the assembly, similar to how the string 2013A may be stored in conventional compact applicator tampon wrappers. As the user pulls the assembly out of the flip top cover 920, the string may come out alongside at least a portion of the tampon applicator.

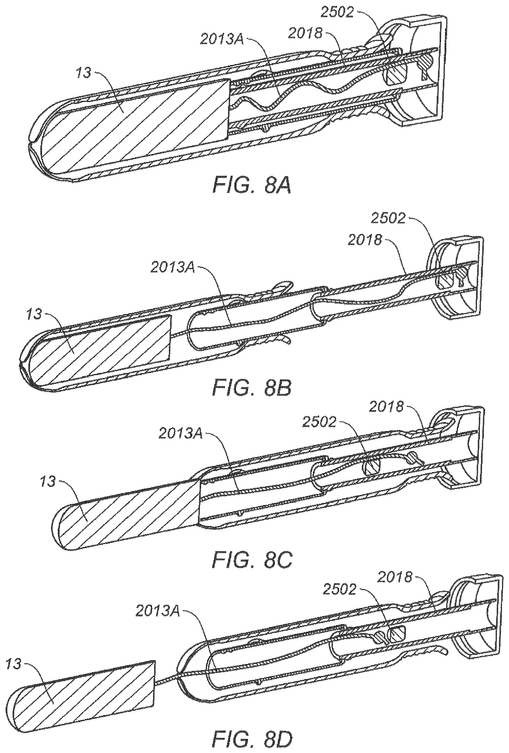

Referring to FIGS. 8A-D, an "obstruction" 2502 (an object that would guide the string 2013A above, below or through it) can be placed in the plunger 2018 which provides slight resistance on the string 2013A during pledget 13 deployment. The obstruction 2502 can be stationary or mobile and remain inside of the plunger 2018 while the string 2013A slides past. The stationary obstruction 2502 can be an integral part of the plunger 2018 (e.g., made together) or attached later directly to the plunger 2018 anywhere along the length of the plunger 2018. The mobile obstruction 2502 can be inserted into the plunger 2018 at any stage during construction of the assembly.

In some embodiments, obstruction 2502 is a compliant material such that (a) during the assembly process, a vacuum can be applied to plunger 2018 and the string 2013A will be able to pass obstruction 2502 such that it is retained by obstruction 2502 at least within a portion of plunger 2018, and (b) during ejection of the pledget, the string 2013A is able to pass obstruction 2502 and exit the plunger 2018 as it is deployed.

The obstruction 2502 can be made from a "soft" material (such as cotton, rayon, paper, etc.) as well as a harder material (e.g., plastic such as polyethylene, polypropylene, etc.). The thickness and material of the obstruction 2502 can be designed such that the obstruction 2502 acts like a diaphragm with a hole that would allow the string 2013A to go through. The obstruction 2502 can fill 0.1% to 99% of the cross-sectional area of the plunger 2018; in some embodiments 50% to 99% if the obstruction 2502 is made from a "soft" material (as a soft obstruction 2502 would give way to the string 2013A) and 1% to 90% (e.g., 1% to 60%) if the obstruction 2502 is made from a "harder" material. The cross-sectional shape of the obstruction 2502 can be circular, rectangular, triangular or a combination thereof. The largest outer diameter of the obstruction 2502 can be between 0.1% and 150% of the string 2013A knot diameter; in some embodiments between 10% and 90%.

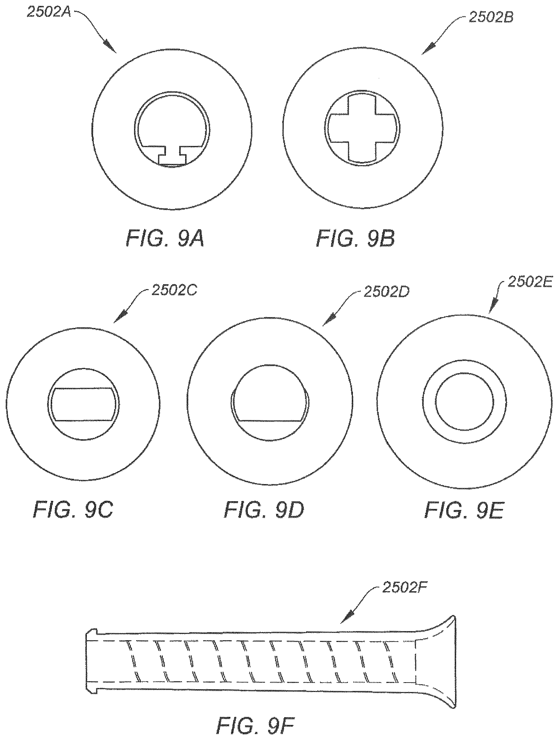

FIGS. 9A-F shows various embodiments for the obstruction 2502. The obstruction 2502 can be in the shape of a "channel" 2502A, "cross-fin" 2502B, "half-moon" 2502C, "throttle" 2502D, "wings" 2502E and "spiral" 2502F, or any combination thereof. As one skilled in the art would appreciate based on a review of this disclosure, the geometry of the obstruction 2502 is not limited to the geometries just described, but can include anything that will act as an obstruction and will guide the string 2013A therethrough. Any such obstructions 2502 can be built into the plunger 2018 or attached to the inside of the plunger 2018 and can be placed anywhere along the length of the plunger 2018. Also, there can be more than one obstruction 2502 along the length of the plunger 2018.

If the obstruction 2502 is in the shape of a channel (or channels) 2502A, the string 2013A can sit in the channel 2502A and can be guided through it during ejection. The number of channels 2502A may be between one and five, e.g., between one and two. The height of the channel 2502A can be 0.1% to 150% of the string 2013A knot 2013B diameter; in some embodiments between 50% and 120% of the string 2013A knot 2013B diameter. Alternatively, the obstruction 2502 can be shaped as cross-fins 2502B. The number of fins 2502B may be between one and ten; in some embodiments between one and four. The distance between the fins 2502B can be from 0.1% to 150%, e.g., from 10%-90% of the string 2013A knot 2013B diameter. If the obstruction 2502 is in the shape of a half-moon 2502C, the height of the moon 2502C can be from 0.1% to 150%, e.g., from 10%-90% of the string 2013A knot 2013B diameter. Alternatively, the obstruction 2502 can be in the shape of a throttle 2502D where the diameter of the plunger 2018 is smaller at a certain point or points along the length of the plunger 2018 that will act like a resistance point. The diameter of the throttle 2502D can be from 0.1% to 150%, e.g., from 10%-90% of the string 2013A knot 2013B diameter. If the shape of the obstruction 2502 is in the form of wings 2502E, the number of wings 2502E may be between one and ten, e.g., between one and four. The distance between the wings 2502D can be from 0.1% to 150%, e.g., from 10%-90% of the string 2013A knot 2013B diameter. The cross-sectional shape of the wings 2502E can be circular, rectangular, triangular, trapezoidal, and elliptical, or any combination thereof. Another alternative is to create spirals or grooves 2502F inside of the plunger 2018 through which the string 2013A may be guided. The size of the spiral or the grooves 2502F can be adjusted such that the string 2013A can freely move through without getting stuck.

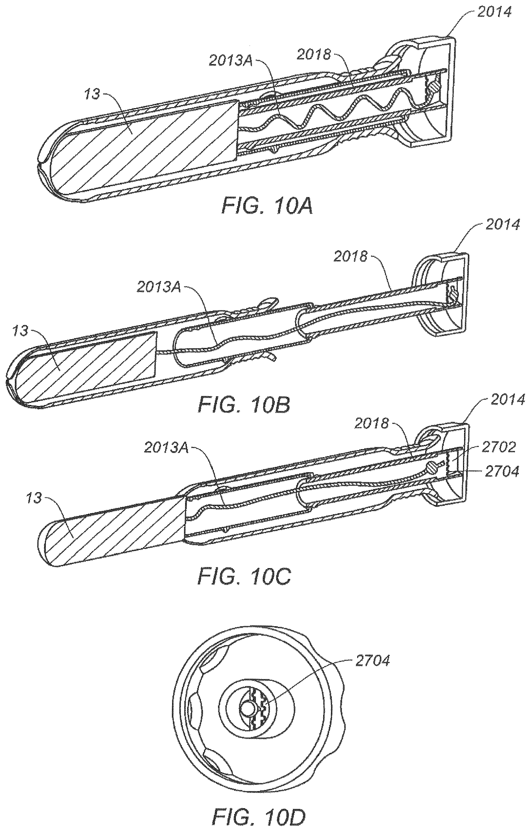

FIGS. 10A-D shows an embodiment that may be used for string 2013A storage and handling. A sticky tab (e.g., adhesive label) 2702 may be applied to the inside of the cap 2014 which retains the string 2013A during pledget 13 deployment. When the string 2013A is fully extended, perforations 2704 in the sticky tab 2702 tear to allow the string 2013A to deploy correctly. The sticky tab 2702 sticks to the cap 2014 but is raised over the string 2013A and thus barely sticks to the string 2013A and is capable of letting the string 2013A go once the pledget 13 is ejected.

The shape of the label 2702 can be circular, rectangular, triangular, trapezoidal, and elliptical, or a combination thereof. The material of the sticky tab 2702 may be made from one or more plastics, paper, paperboard, cardboard, textile, or any combination thereof. The strength of the adhesive on the sticky tab 2702 can be adjusted such that the sticky tab sticks to the cap 2014 material strongly and doesn't lift but releases the string 2013A material which can be cotton, rayon, or a combination thereof. The perforations 2704 on the sticky tab 2702 can be designed such that the sticky tab 2702 tears at the perforation 2704 easily without any extra resistance to the user as she's inserting the product. The sticky tab 2702 can be attached to the string 2013A end which is then attached onto the cap 2014 which is snapped onto the plunger 2018. Alternatively, the sticky tab 2702 may be attached to the cap 2014 first followed by the string 2013A end being tucked under the label 2702. The cap 2014 then can be snapped onto the plunger 2018. Yet alternatively, the end of the string 2013A can be dipped into a light adhesive to attach to the cap 2014 directly which will eliminate this need for a separate sticky tab 2702.

The amount of adhesive can be adjusted such that the string 2013A is loosely attached to the cap 2014 when the product is not in use and easily separates from the cap 2014 when the pledget 13 is inserted into the user's body. Alternatively, the end of string 2013A can be electrostatically charged and attached to the cap 2014 directly using electrostatic forces. Just like the string 2013A end dipped in adhesive, this will eliminate the need for a separate label 2702. The electrostatic forces can be adjusted such that the string 2013A is loosely attached to the cap 2014 when the product is not in use and easily separates from the cap 2014 when the product is inserted into the user's body.

In some embodiments, and potentially in lieu of a use of the sticky tab 2702, hook and loop fasteners such as VELCRO can be applied to the inside of the cap 2014 which retains the string 2013A during pledget 13 deployment. The interaction between the VELCRO and the end of string 2013A may create a minimal, predetermined amount of resistance to hold the string 2013A in place during deployment but release the string 2013A once the pledget 13 is fully ejected. The shape of the VELCRO can be circular, rectangular, triangular, trapezoidal, and elliptical or a combination thereof. The size of the VELCRO applied can be adjusted to create any required forces. Just like the adhesive label 2702, the VELCRO can be attached to the string 2013A end which is then attached onto the cap 2014 which is snapped onto the plunger 2018. Alternatively, the VELCRO may be attached to the cap 2014 first followed by the string 2013A end being tucked under the VELCRO. The cap 2014 then can be snapped onto the plunger 2018.

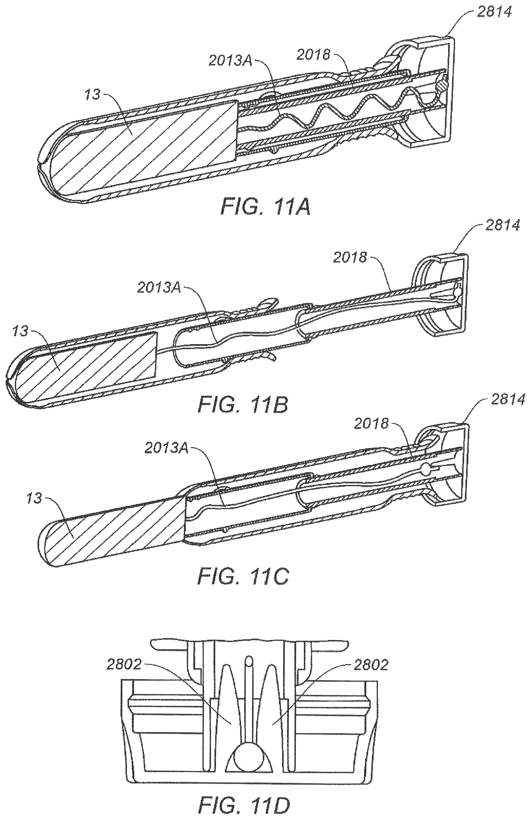

FIGS. 11A-D illustrate an embodiment including a cap 2814 where the string 2013A end is held on the cap 2814 until the string 2013A is pulled during pledget 13 deployment/ejection. In particular, FIGS. 11A-D shows a "tab" embodiment where the end of string 2013A (above or below the knot) is held in place by an opening between tabs 2802. However, the tabs 2802 can be of any shape, such as circular, rectangular, triangular, trapezoidal, and elliptical or a combination thereof. The number of tabs 2802 may be between one and ten, e.g., between two and five. The distance between the tabs 2802 can be 0.1% to 150%, e.g., from 10%-90% of the string 2013A knot diameter. The larger opening between the tabs 2802 can be 0.1% to 150%, e.g., between 30%-110% of the string 2013A knot diameter. The tabs 2802 can be manufactured separately and attached to the cap 2814 by any chemical (adhesive etc.) or mechanical technique (living hinge etc.) or a combination thereof (ultrasonic bonding etc.). In some embodiments, the tabs 2802 may be manufactured together with the cap 2814. After the tabs 2802 are attached to the cap 2814, the string 2013A can be tucked in between in the tabs 2802 and the cap 2814 is snapped onto the plunger 2018. Alternatively, the string 2013A can be tucked in between the tabs 2802 which are then attached to the cap 2814 which is then snapped onto the plunger 2018. There may be a hole 2021 on the other side of the cap 2814 to allow for a vacuum to suck the string 2013A through the plunger 2018 during assembly. The hole 2021 size can be between 1% and 150% of the string 2013A knot 2013B size, e.g., between 20% and 70%. The hole 2021 can then be closed by using a sticky tab or attaching another part by ultrasonic welding or any other attachment techniques.

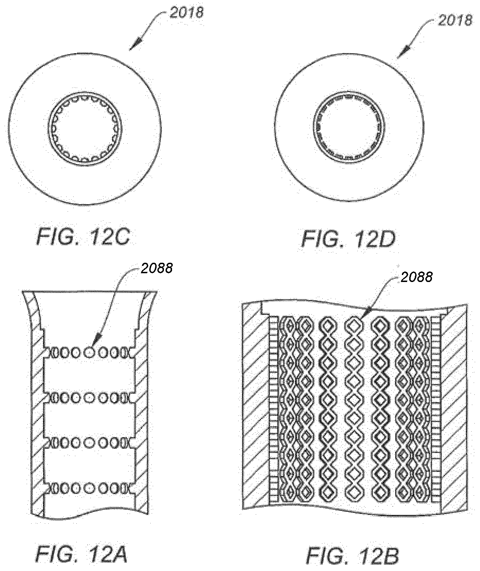

FIGS. 12A-D illustrates a use of a textured surface 2088 inside of the plunger 2018, such that a larger surface area is created that is in contact with the string 2013A. As the string 2013A (especially the string end) touches the textured surface 2088, the intermolecular forces can be large enough to hang onto the string 2013A in a sufficient, minimal amount such that a resistance is generated and the string 2013A untangles as the string 2013A travels through the plunger 2018. The intermolecular forces can be adjusted by adjusting the amount of texturing (the texture can be applied at a certain spot or all throughout the inner surface of the plunger 2018); the texture pattern (such as spikes or bumps as generally represented in FIGS. 12A and 12C, and diamonds as generally represented in FIGS. 12B and 12D, or any other pattern); the height of the pattern (the texture can be small, e.g., micron level or less, or large) and the material of the textured surface 2088 (the inside of the plunger 2018 can be made from a high friction material). If the inside of the plunger 2018 is made from a high friction material (such as a thermoplastic elastomer (TPE) or high durometer plastic), the material itself might be adequate enough to create the forces and no textured surface 2088 might be needed.

In some embodiments, the string 2013A material may include cotton, rayon, or a combination thereof which can "set-in" (wrinkle, tangle and take the shape of the position that they're in for an extended amount of time) as they're pretty flexible. Alternatively, a more "rigid" string 2013A material can be used that won't set-in like cotton and rayon and that will "bounce back" to its original shape right after the restriction (in this case being enclosed in the plunger 2018 for a while) is removed. This enables the string 2013A to not stay bunched up but untangle by itself and get back to its original shape right after the pledget 13 is ejected.

Conventional tampon strings may have a single knot towards the end of the string to enable an easier grasp point for the user. More than one knot can be placed on the string 2013A, such that it makes it more difficult for the string 2013A to set-in and helps the string 2013A to bounce back to its original shape upon insertion. The knot type can be changed to a bowline-like knot that will enable a big loop in the middle of the knot 2013B so that the string 2013A would untangle easier as it moves through the plunger 2018. The end of the string 2013A can be combed to increase the amount of interaction between the string 2013A and the sticky tab (e.g., FIG. 10 A-D as described above), string and the VELCRO as described above, and string and the texturing (e.g., FIG. 12 A-D as described above).

Referring now to FIGS. 13A-D, cap 3014 has retention structures 3011 that resist the force exerted on tampon applicator package assembly 3010 as the user draws the telescoping plunger 3018 rearward from the applicator barrel 3016. In some embodiments, telescoping plunger 3018 is a two-piece plunger having an outer plunger 3087 that engages barrel 3016 and inner plunger 3085 that engages outer plunger 3087. Inner plunger 3085 and outer plunger 3087 connect via a connection mechanism 3086, such as a detent mechanism, a flexible latch mechanism, a friction fit, and combinations thereof. In an initial state as shown in FIG. 13A, inner plunger 3085 is telescoped within outer plunger 3087 such that the tampon applicator package assembly is in a compact form. After opening the cap 3014 by moving the cap cover 3083 to reveal the tampon applicator (e.g., at least one of the plunger 3018 and the string 3013A), As the user draws the two-piece plunger rearward, a force of less than about 13 N is required to assemble the inner plunger 3085 and outer plunger 3087 in a fully-extended state. In some embodiments, a force of less than 8 N is required to assemble inner plunger 3085 and outer plunger 3087 in a fully-extended state. In further embodiments, a force of between about 3 N to about 7.5 N is required to assemble inner plunger 3085 and outer plunger 3087 in a fully-extended state. More preferably, the force required to assemble inner plunger 3085 and outer plunger 3087 in a fully-extended state is between about 4 N and about 6 N. The retention structures 3011 must cooperate with the applicator barrel 3016 (and in some embodiments, the grip region 3081) to ensure the applicator barrel 3016 is not advanced rearward out of the cap 3014 while the user is either (a) drawing the plunger 3018 rearward such that it is in a fully-extended state, or (b) assembling the inner plunger 3087 and outer plunger 3085 into a fully-extended state. In other words, the retention force (e.g., the force exerted by retention structures 3011 on the applicator barrel 3016) is greater than the force to prepare the plunger in a fully-extended state. In some embodiments, the retention force is greater than about 13 N. In further embodiments, the retention force is greater than about 10 N. In further embodiments, the retention force is greater than about 8 N. Most preferably, the retention force is greater than the force to prepare the plunger in a fully-extended state, but is not difficult to achieve by the consumer. As such, in preferred embodiments, the retention force is between about 8 N and about 10 N.

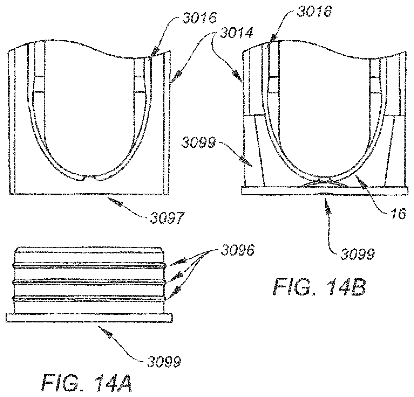

FIGS. 14A and 14B show a further embodiment of the present disclosure that is advantageous from an assembly standpoint. FIG. 14A shows an exploded view of a plug 3099 and cap 3014 having retention structures 3099 in the form of ribs. FIG. 14B shows an assembled view of plug 3099 and cap 3014 having retention structures 3099. Retention structures 3099 must provide a sufficiently tight closure with cap 3014 while also mitigating the amount of interference with insertion end 16 the applicator 3016. During assembly, applicator barrel 3016 is inserted via orifice 3097 and thereafter, plug is press-fit into orifice 3097 to sufficiently seal orifice 3097, thereby mitigating against any moisture and/or other foreign material from reaching the contents therein. Having such an assembly is advantageous as it allows assembly of the applicator barrel 3016 into the cap 3014 and thereby avoids further complications with keeping string 3018 in an orderly state, as described throughout the present disclosure.

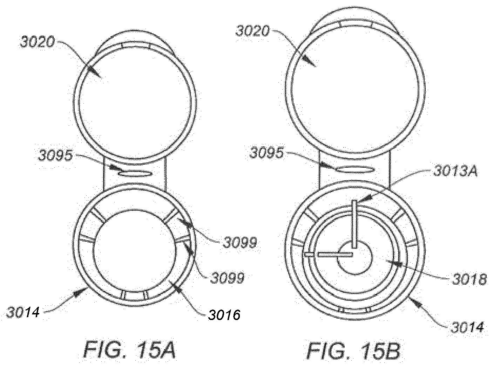

FIGS. 15A-B show a further embodiment of a cap 3014 wherein the retention structures 3099 are eccentric such that the applicator barrel 3016 is other than concentrically located within the cap 3014. Eccentric retention provides a larger space for the string 3013A and the knot 2013B and thereby reduces tangling during assembly and during removal of the applicator by the user during use. Eccentric retention further provides greater accessibility to the plunger 3018 as a larger gap is on one side of the applicator barrel 3016 (e.g., the larger gap provides a larger space for the user to slide her finger adjacent the applicator and/or plunger 3018 to assist in extending the plunger 3018 and thereafter removing the applicator barrel 3016 from cap 3014). Hinge 3095 is also advantageous in that it provides snap-action for the cap cover 3020 to open and close with respect to cap 3014. As the user opens cap cover 3020 and rotates it backward, hinge 3095 with is ovular recessed central portion acts as a spring and assists in deflecting cap cover 3020 away from the open end of cap 3014 and thereby providing access to the applicator, plunger 3018 and/or string 3013A.

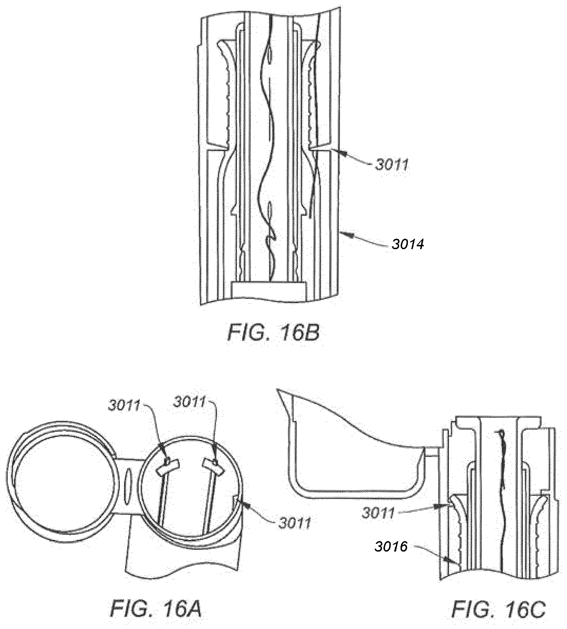

FIGS. 16A-C show a further embodiment where retention structures 3099 are flexible. These flexible retention structures shaped similar to tabs provide a sufficient retention force as described throughout the present disclosure. Furthermore, the flexible tabs are less likely to leave blemishes or marks on the applicator barrel 3014 as it is removed from the cap 3014.

It should also be noted that the terms "first", "second", "third", "upper", "lower", and the like may be used herein to modify various elements. These modifiers do not imply a spatial, sequential, or hierarchical order to the modified elements unless specifically stated.

**Claims**

1. A tampon applicator package assembly with a tampon assembly, comprising:

   a container, comprising:

a body (3014) forming a cavity for holding the tampon assembly, the body (3014) having an interior wall with a plurality of retention structures (3011) extending outward from the body (3014) into the cavity; and a cap cover (3083, 3020, 920) coupled to the body (3014) configured to enclose the tampon assembly within the body (3014) when the cap cover (3020, 3083, 920) is in a first position and provide access to the tampon assembly when the cap cover (3083, 3020, 920) is in a second position; and

a tampon assembly comprising:

a tampon applicator having an insertion region, a barrel region (3016) adjacent the insertion region, and a grip region (3081) adjacent the barrel region (3016), the barrel region (3016) defining a hollow volume; and a tampon pledget having an insertion end and a withdrawal end, the withdrawal end having a string (3013A) attached thereto, the tampon pledget located within the barrel region (3016) such that the string (3013A) extends into and through the grip region; and a two-piece telescoping plunger (3018) having an inner plunger (3085) that engages an outer plunger (3087), wherein the inner plunger (3085) and the outer plunger (3087) engage by a connection mechanism (3086), wherein the outer plunger (3087) engages the barrel region (3016), wherein the inner plunger (3085) is fully telescoped within the outer plunger (3087) when the tampon assembly is within the body (3014) of the container of the tampon applicator package assembly when the cap cover (3020, 3083, 920) is in the first position:
wherein the retention structures (3011) engage the tampon applicator about at least one of the insertion region, barrel region (3016) and grip region,
wherein a connection mechanism rearward force as the user draws the telescoping plunger (3018) rearward from the applicator barrel (3016) is required for the outer plunger (3087) to engage the inner plunger (3085) such that the outer plunger (3087) and the inner plunger (3085) are connected in a fully-extended state,
wherein a retention structure force exerted by the retention structures (3011) is greater than the connection mechanism force.

2. The tampon applicator package assembly according to the preceding claim, wherein said container further comprises a plug (3099) that engages a first end of the container.

3. The tampon applicator package assembly according to any of the preceding claims, wherein said retention structures (3011) are ribs.

4. The tampon applicator package assembly according to any of the preceding claims, wherein said retention structures (3011) are eccentrically positioned such that the tampon assembly is eccentrically positioned within the container volume.

5. The tampon applicator package assembly according to claim 4, wherein the string (3013A) is positioned in the larger open portion of the container volume.

6. The tampon applicator package assembly according to any of the preceding claims, wherein the connection mechanism (3086) is at least one of a detent mechanism, a flexible latch mechanism and a friction fit.

7. The tampon applicator package assembly according to claim 1, wherein the connection mechanism force is less than 13 N.

8. The tampon applicator package assembly according to claim 1, wherein the connection mechanism force is less than 8 N.

9. The tampon applicator package assembly according to claim 1, wherein the connection mechanism force is between 3 N and 7.5 N.

10. The tampon applicator package assembly according to claim 1, wherein the retention structure force is greater than 8 N.

11. The tampon applicator package assembly according to claim 1, wherein the retention structure force is between 8 N and 10 N.

**12.** The tampon applicator package assembly according to claim 1, wherein the retention structure force is greater than 13 N.

**Patentansprüche**

**1.** Tamponapplikator-Verpackungsanordnung mit einer Tamponanordnung, die aufweist:

einen Behälter, der aufweist:

einen Körper (3014), der einen Hohlraum zum Halten der Tamponanordnung bildet, wobei der Körper (3014) eine Innenwand mit einer Vielzahl von Rückhaltestrukturen (3011) aufweist, die sich von dem Körper (3014) nach außen in den Hohlraum erstrecken; und
eine Kappenabdeckung (3083, 3020, 920), die mit dem Körper (3014) gekoppelt ist und dazu ausgebildet ist, die Tamponanordnung in dem Körper (3014) zu umgeben, wenn die Kappenabdeckung (3020, 3083, 920) in einer ersten Position ist, und Zugang zur Tamponanordnung zu ermöglichen, wenn die Kappenabdeckung (3083, 3020, 920) in einer zweiten Position ist; und

eine Tamponanordnung, die aufweist:

einen Tamponapplikator mit einem Einführungsbereich, einem Hülsenbereich (3016) benachbart zum Einführungsbereich, und einen Griffbereich (3081) benachbart zum Hülsenbereich (3016), wobei der Hülsenbereich (3016) ein Hohlvolumen definiert; und
einen Tamponbausch mit einem Einführungsende und einem Entnahmeende, wobei das Entnahmeende einen daran angebrachten Faden (3013A) aufweist, wobei der Tamponbausch sich in dem Hülsenbereich (3016) befindet, so dass sich der Faden (3013A) in und durch den Griffbereich erstreckt; und
einen zweiteiligen teleskopierenden Kolben (3018) mit einem inneren Kolben (3085), der mit einem äußeren Kolben (3087) zusammengreift,
wobei der innere Kolben (3085) und der äußere Kolben (3087) durch einen Verbindungsmechanismus (3086) zusammengreifen,
wobei der äußere Kolben (3087) den Hülsenbereich (3016) angreift,
wobei der innere Kolben (3085) vollständig in dem äußeren Kolben (3087) teleskopiert ist, wenn sich die Tamponanordnung in dem Körper (3014) des Behälters der Tamponapplikator-Verpackungsanordnung befindet, wenn die Kappenabdeckung (3020, 3083, 920) in der ersten Position ist,

wobei die Rückhaltestrukturen (3011) den Tamponapplikator um mindestens eines aus dem Einführungsbereich, dem Hülsenbereich (3016) und dem Griffbereich angreifen,
wobei eine nach hinten gerichtete Kraft des Verbindungsmechanismus, wenn der Benutzer den teleskopierenden Kolben (3018) nach hinten aus der Applikatorhülse (3016) zieht, erforderlich ist, damit der äußere Kolben (3087) mit dem inneren Kolben (3085) zusammengreift, so dass der äußere Kolben (3087) und der innere Kolben (3085) in einem vollständig ausgefahrenen Zustand verbunden sind,
wobei eine Rückhaltestrukturkraft, die von den Rückhaltestrukturen (3011) ausgeübt wird, größer ist als die Verbindungsmechanismuskraft.

**2.** Tamponapplikator-Verpackungsanordnung nach dem vorstehenden Anspruch, wobei der Behälter ferner einen Stopfen (3099) aufweist, der mit einem ersten Ende des Behälters zusammengreift.

**3.** Tamponapplikator-Verpackungsanordnung nach einem der vorstehenden Ansprüche, wobei die Rückhaltestrukturen (3011) Rippen sind.

**4.** Tamponapplikator-Verpackungsanordnung nach einem der vorstehenden Ansprüche, wobei die Rückhaltestrukturen (3011) exzentrisch positioniert sind, so dass die Tamponanordnung exzentrisch in dem Behältervolumen positioniert ist.

**5.** Tamponapplikator-Verpackungsanordnung nach Anspruch 4, wobei der Faden (3013A) in dem größeren offenen Abschnitt des Behältervolumens positioniert ist.

**6.** Tamponapplikator-Verpackungsanordnung nach einem der vorstehenden Ansprüche, wobei der Verbindungsmechanismus (3086) mindestens eines aus einem Rastmechanismus, einem flexiblen Riegelmechanismus und einer Reibungspassung ist.

7. Tamponapplikator-Verpackungsanordnung nach Anspruch 1, wobei die Verbindungsmechanismuskraft weniger als 13 N beträgt.

8. Tamponapplikator-Verpackungsanordnung nach Anspruch 1, wobei die Verbindungsmechanismuskraft weniger als 8 N beträgt.

9. Tamponapplikator-Verpackungsanordnung nach Anspruch 1, wobei die Verbindungsmechanismuskraft zwischen 3 N und 7,5 N beträgt.

10. Tamponapplikator-Verpackungsanordnung nach Anspruch 1, wobei die Rückhaltestrukturkraft mehr als 8 N beträgt.

11. Tamponapplikator-Verpackungsanordnung nach Anspruch 1, wobei die Rückhaltestrukturkraft zwischen 8 N und 10 N beträgt.

12. Tamponapplikator-Verpackungsanordnung nach Anspruch 1, wobei die Rückhaltestrukturkraft mehr als 13 N beträgt.

## Revendications

1. Ensemble d'emballage d'applicateur de tampon avec un ensemble de tampon, comprenant :
un contenant, comprenant :

un corps (3014) formant une cavité pour contenir l'ensemble de tampon, le corps (3014) ayant une paroi intérieure avec une pluralité de structures de rétention (3011) s'étendant vers l'extérieur depuis le corps (3014) jusque dans la cavité ; et
un couvercle de capuchon (3083, 3020, 920) couplé au corps (3014) configuré pour enfermer l'ensemble de tampon au sein du corps (3014) lorsque le couvercle de capuchon (3020, 3083, 920) est dans une première position et permettre l'accès à l'ensemble de tampon lorsque le couvercle de capuchon (3083, 3020, 920) est dans une seconde position ; et
un ensemble de tampon comprenant :

un applicateur de tampon ayant une région d'insertion, une région de cylindre (3016) adjacente à la région d'insertion, et une région de préhension (3081) adjacente à la région de cylindre (3016), la région de cylindre (3016) définissant un volume creux ; et
une compresse de tampon ayant une extrémité d'insertion et une extrémité de retrait, l'extrémité de retrait ayant une ficelle (3013A) attachée à celle-ci, la compresse de tampon étant située au sein de la région de cylindre (3016) de telle sorte que la ficelle (3013A) s'étend dans et à travers la région de préhension ; et
un piston télescopique en deux parties (3018) ayant un piston interne (3085) qui engage un piston externe (3087),
dans lequel le piston interne (3085) et le piston externe (3087) s'engagent par un mécanisme de raccordement (3086),
dans lequel le piston externe (3087) engage la région de cylindre (3016),
dans lequel le piston interne (3085) est entièrement télescopé au sein du piston externe (3087) lorsque l'ensemble de tampon est au sein du corps (3014) du contenant de l'ensemble d'emballage d'applicateur de tampon lorsque le couvercle du capuchon (3020, 3083, 920) est dans la première position ;
dans lequel les structures de rétention (3011) engagent l'applicateur de tampon autour d'au moins une parmi la région d'insertion, la région de cylindre (3016) et la région de préhension,
dans lequel une force arrière de mécanisme de raccordement lorsque l'utilisateur tire le piston télescopique (3018) vers l'arrière depuis le corps de l'applicateur (3016) est nécessaire pour que le piston externe (3087) engage le piston interne (3085) de telle sorte que le piston externe (3087) et le piston interne (3085) sont raccordés dans un état complètement étendu,
dans lequel une force de structure de rétention exercée par les structures de rétention (3011) est supérieure à la force de mécanisme de raccordement.

**2.** Ensemble d'emballage d'applicateur de tampon selon la revendication précédente, dans lequel ledit contenant comprend en outre un bouchon (3099) qui engage une première extrémité du contenant.

**3.** Ensemble d'emballage d'applicateur de tampon selon l'une quelconque des revendications précédentes, dans lequel lesdites structures de rétention (3011) sont des nervures.

**4.** Ensemble d'emballage d'applicateur de tampon selon l'une quelconque des revendications précédentes, dans lequel lesdites structures de rétention (3011) sont positionnées de manière excentrique, de telle sorte que l'ensemble de tampon est positionné de manière excentrique au sein du volume de contenant.

**5.** Ensemble d'emballage d'applicateur de tampon selon la revendication 4, dans lequel la ficelle (3013A) est positionnée dans la portion ouverte la plus large du volume de contenant.

**6.** Ensemble d'emballage d'applicateur de tampon selon l'une quelconque des revendications précédentes, dans lequel le mécanisme de raccordement (3086) est au moins l'un parmi un mécanisme d'encliquetage, un mécanisme de verrouillage flexible et un ajustement par frottement.

**7.** Ensemble d'emballage d'applicateur de tampon selon la revendication 1, dans lequel la force de mécanisme de raccordement est inférieure à 13 N.

**8.** Ensemble d'emballage d'applicateur de tampon selon la revendication 1, dans lequel la force de mécanisme de raccordement est inférieure à 8 N.

**9.** Ensemble d'emballage d'applicateur de tampon selon la revendication 1, dans lequel la force de mécanisme de raccordement est entre 3 N et 7,5 N.

**10.** Ensemble d'emballage d'applicateur de tampon selon la revendication 1, dans lequel la force de structure de rétention est supérieure à 8 N.

**11.** Ensemble d'emballage d'applicateur de tampon selon la revendication 1, dans lequel la force de structure de rétention est entre 8 N et 10 N.

**12.** Ensemble d'emballage d'applicateur de tampon selon la revendication 1, dans lequel la force de structure de rétention est supérieure à 13 N.

FIG. 1A

FIG. 1B

FIG. 1C

FIG. 1D

FIG. 2

EP 3 474 801 B1

2020

2019

2019

2014A

2013A

2014

2018

*FIG. 3B*

*FIG. 3A*

FIG. 3C      FIG. 3D      FIG. 3E

FIG. 3F

FIG. 3G

FIG. 3H

FIG. 3I

FIG. 3J

FIG. 3K

FIG. 3L

FIG. 3M

FIG. 3N

FIG. 3O

FIG. 4A

FIG. 4B

FIG. 5E

FIG. 5D

FIG. 5C

FIG. 5B

FIG. 5A

FIG. 6

920

2013A

2420

2013A

920

2420

2414A

2430

2414A

*FIG. 7A*

*FIG. 7B*

FIG. 8A

FIG. 8B

FIG. 8C

FIG. 8D

2502A

2502B

FIG. 9A

FIG. 9B

2502C

2502D

2502E

FIG. 9C

FIG. 9D

FIG. 9E

2502F

FIG. 9F

FIG. 10A

FIG. 10B

FIG. 10C

FIG. 10D

FIG. 11A

FIG. 11B

FIG. 11C

FIG. 11D

2018

FIG. 12C

2018

FIG. 12D

2088

FIG. 12A

2088

FIG. 12B

FIG. 13A

FIG. 13B

FIG. 13C

FIG. 13D

*3016*

*3016*

*3014*

*3099*

*3099*

*16*

*3097*

*3096*

*3099*

FIG. 14B

FIG. 14A

FIG. 15A

FIG. 15B

*3011*

*3014*

FIG. 16B

*3011*   *3011*

*3011*

FIG. 16A

*3011*

*3016*

FIG. 16C

**REFERENCES CITED IN THE DESCRIPTION**

**Patent documents cited in the description**

- WO 2014004560 A
- WO 2017015426 A
- EP 3324909 A
- WO 2010120282 A
- WO 0119310 A
- US 5988386 A